# EUROPÄISCHE PATENTSCHRIFT

(11) **EP 0 485 928 B1**
(45) Veröffentlichungstag und Bekanntmachung des Hinweises auf die Patenterteilung: **01.02.1995**
(21) Anmeldenummer: 91119185.6
(22) Anmeldetag: 11.11.1991
(51) Int. Cl.: C07C 409/40, C07C 407/00, C11D 3/39, A01N 47/28, A01N 47/30

(54) **Verfahren zur Herstellung von Ureidoperoxicarbonsäuren**
Process for the preparation of ureidoperoxycarboxylic acids
Procédé pour la préparation d'acides ureidoperoxycarboxyliques

(30) Priorität: 16.11.1990 DE 4036646
(43) Veröffentlichungstag der Anmeldung: 20.05.1992
(73) Patentinhaber: HOECHST AKTIENGESELLSCHAFT, 65926 Frankfurt am Main (DE)
(72) Erfinder: Müller, Wolf-Dieter, Dr., W-6238 Hofheim am Taunus (DE); Jaekel, Frank, Dr., W-6232 Bad Soden (Taunus) (DE); Naumann, Peter, Dr., W-6204 Taunusstein (DE); Reinhardt, Gerd, Dr., W-6233 Kelkheim (Taunus) (DE)

(56) Entgegenhaltungen:
- EP-A- 0 349 940
- EP-A- 0 458 327
- NL-A- 6 717 087

## Beschreibung

Die vorliegende Erfindung bezieht sich auf ein Verfahren zur Herstellung von Ureidoperoxicarbonsäuren der allgemeinen Formel

A⁅NH - CO - NH - B - CO - OOH]ₓ ,

worin
- x: die Zahlen 1 oder 2,
- A: falls x = 1 ist,
Wasserstoff, C₁-C₂₀-Alkyl, Aryl, vorzugsweise Phenyl, oder Halogenaryl, vorzugsweise Chlorphenyl,
falls x = 2 ist,
C₁-C₂₀-Alkylen oder Arylen, vorzugsweise Phenylen, bedeuten,
- B: eine Gruppe der Formeln 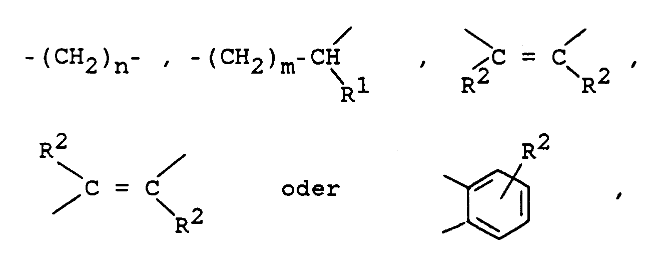 worin
- n: die Zahlen von 1 bis 20,
- m: die Zahlen 0, 1 oder 2,
- R¹: C₁-C₂₀-Alkyl und
- R²: jeweils Wasserstoff oder C₁-C₂₀-Alkyl bedeuten.

Ureidoperoxicarbonsäuren sind Oxidationsmittel und können zur Bleiche von Textilien und zur Desinfektion, insbesondere in sanitären Anlagen, verwendet werden, da ihre Wirkung bereits bei Temperaturen von 60°C und darunter eintritt (Deutsche Patentanmeldung P 40 16 980.4). Die Synthese der Ureidocarbonsäuren, die Vorläufer der analogen Peroxicarbonsäuren, kann aus einem Isocyanat und einer ω-Aminocarbonsäure erfolgen (Deutsche Patentanmeldung P 40 16 980.4). Verfahrensbedingt entsteht dabei pro Mol Ureidocarbonsäure ein Mol Salz. Die sich anschließende Oxidation zu den entsprechenden Persäuren geschieht wie in EP-A-349 940 beschrieben.

Ein anderes Verfahren geht von Isocyanaten und Lactamen aus, aus denen zunächst Carbamoyllactame dargestellt werden. Carbamoyllactame an sich finden als verkappte Isocyanate eine breite Anwendung und sind gut und kostengünstig zugänglich (EP-A-10 766, EP-A-23 649, BE-A-739 313). Eine Öffnung des Lactamringes unter basischen Bedingungen, beispielsweise mit Natriumhydroxid, führt zwangsläufig zum unerwünschten Salzanfall und ist daher für die großtechnische Anwendung ebenfalls ungeeignet. Spaltungen mit Aminen verlaufen jedoch unselektiv und ergeben Produktgemische (Arch. Pharm. 320 (1987), 430). Eine Öffnung des Lactamringes im Carbamoyllactam mit Natriummethylat ist in DE-A-35 04 967 beschrieben und verläuft zwar selektiv, aber naturgemäß unter Salzbildung.

Der vorliegenden Erfindung liegt die Aufgabe zugrunde, ein Verfahren zur Herstellung von Ureidoperoxicarbonsäuren zur Verfügung zu stellen, das ökologisch unbedenklich ist und bei dem preiswerte Ausgangsprodukte eingesetzt werden können.

Es wurde überraschend gefunden, daß die Hydrolyse der Carbamoyllactame unter sauren Bedingungen einen eindeutigen Raktionsverlauf nimmt und in hoher Ausbeute und Reinheit die Ureidocarbonsäuren liefert. Darüberhinaus können diese Ureidocarbonsäuren unter den angewandten Reaktionsbedingungen mit Wasserstoffperoxid direkt, d.h. in einer Eintopfreaktion, zu den entsprechenden Persäuren oxidiert werden.

Die Aufgabe wird dadurch gelöst, daß Carbamoyllactame der allgemeinen Formel 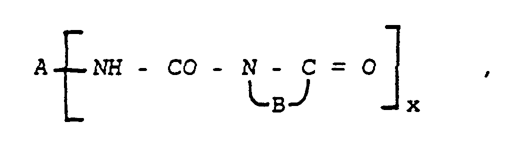
worin
- x: die Zahlen 1 oder 2,
- A: falls x = 1 ist,
Wasserstoff, C₁-C₂₀-Alkyl, Aryl, vorzugsweise Phenyl, oder Halogenaryl, vorzugsweise Chlorphenyl,
falls x = 2 ist,
C₁-C₂₀-Alkylen oder Arylen, vorzugsweise Phenylen, bedeuten und
- B: eine Gruppe der Formeln 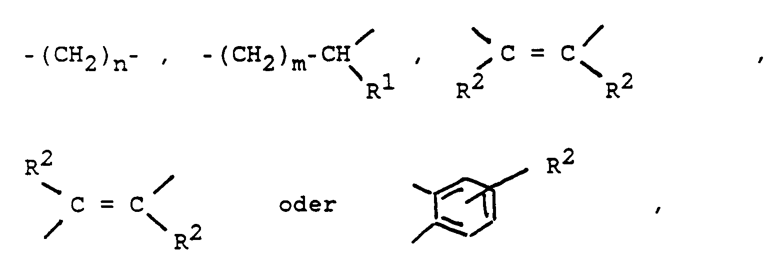 worin
- n: die Zahlen von 1 bis 20,
- m: die Zahlen 0, 1 oder 2,
- R¹: C₁-C₂₀-Alkyl und
- R²: jeweils Wasserstoff oder C₁-C₂₀-Alkyl, bedeuten,
in der ein- bis sechsfachen, vorzugsweise ein- bis dreifachen, Gewichtsmenge einer starken Katalysatorsäure gelöst werden, daß danach zu der Lösung des Carbamoyllactams in der Katalysatorsäure Wasser in ein- bis zehnfachem molarem Überschuß, bezogen auf das Carbamoyllactam, zugegeben und das Reaktionsgemisch zur Bildung der Ureidocarbonsäure erwärmt wird, daß anschließend eine wäßrige Wasserstoffperoxid-Lösung im ein- bis zehnfachen molaren Überschuß pro oxidierbarer Carboxylgruppe der im vorhergehenden Reaktionsschritt entstandenen Ureidocarbonsäure dem Reaktionsgemisch zugesetzt wird, und daß danach die entstandene Ureidoperoxicarbonsäure aus dem Reaktionsgemisch ausgefällt wird.

Als starke Katalysatorsäuren werden beispielsweise Schwefelsäure, Methansulfonsäure oder ein saurer Ionenaustauscher verwendet. Vorzugsweise wird Schwefelsäure als 50 bis 96 gew.-%ige, insbesondere als 75 bis 96 gew.-%ige, wäßrige Lösung eingesetzt. Zweckmäßigerweise wird das Carbamoyllactam zunächst in der 1- bis 6-fachen, vorzugsweise der 1- bis 3-fachen, Gewichtsmenge, bezogen auf das Carbamoyllactam, an Katalysatorsäure gelöst. Um den oder die Lactamringe des Carbamoyllactams hydrolytisch zu öffnen, wird zu der Lösung des Carbamoyllactams in der obengenannten Katalysatorsäure Wasser im ein- bis 10-fachen, vorzugsweise ein- bis dreifachen, molaren Überschuß pro Lactamring zugesetzt und das Reaktionsgemisch für mindestens 10, vorzugsweise 10 bis 120 Minuten, insbesondere 30 bis 80 Minuten, auf Temperaturen von mindestens 40, vorzugsweise 40 bis 120°C, insbesondere 50 bis 80°C erwärmt.

Die aus der Umsetzung des Carbamoyllactams mit Säure und Wasser entstandene Ureidocarbonsäure kann in dem vorliegenden Reaktionsgemisch direkt zur Ureidoperoxicarbonsäure oxidiert werden, ohne daß es notwendig wäre, die Ureidocarbonsäure vor der Oxidation zu isolieren. Für die Oxidation wird Wasserstoffperoxid pro oxidierbarer Carboxylgruppe der Ureidocarbonsäure im ein- bis zehnfachen, vorzugsweise zwei- bis vierfachen, molaren Überschuß dem Reaktionsgemisch zugesetzt. Zweckmäßigerweise verwendet man dabei Wasserstoffperoxid als 5 bis 95 gew.-%ige, vorzugsweise 25 bis 85 gew.-%ige, wäßrige Lösung. Die Reaktionstemperatur und Reaktionszeit für die Oxidation richten sich nach der Stabilität der entstehenden Ureidoperoxicarbonsäure. Die Reaktionstemperatur liegt zwischen -20 und +80°C, vorzugsweise zwischen 5 und 60°C, insbesondere zwischen 15 und 50°C, die Reaktionszeit zwischen 15 und 180 Minuten, vorzugsweise zwischen 20 und 120 Minuten.

Die Ureidoperoxicarbonsäure fällt im allgemeinen durch Zugabe von Wasser aus dem Reaktionsgemisch aus und kann durch Filtration oder Zentrifugation isoliert werden. Es ist auch möglich, Ureidoperoxicarbonsäuren, die durch Wasserzugabe nicht oder nur unvollständig ausfallen, durch Zugabe von wäßrigen Lösungen basischer Salze auszufällen.

Die für das erfindungsgemäße Verfahren als Ausgangsprodukte benötigten Carbamoyllactame können nach bekannten Verfahren entweder in einem organischen Lösungsmittel (EP-A-23 649 und DE-A-33 22 722) oder lösungsmittelfrei (DE-A-31 43 060 und DE-A-35 36 017) aus einem Isocyanat der allgemeinen Formel A-[NCO]ₓ und einem Lactam der allgemeinen Formel 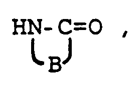
wobei A, B und x die obengenannten Bedeutungen haben, hergestellt werden. Als Isocyanate werden vorzugsweise Phenylisocyanat, p-Tolylisocyanat, Cyclohexylisocyanat, Octylisocyanat und Hexamethylendiisocyanat und als Lactame vorzugsweise Laurinlactam, ε-Caprolactam und Pyrrolidon verwendet.

Als vorteilhaft für die Zwecke der Erfindung haben sich folgende Ureidoperoxicarbonsäuren erwiesen:
N-(N'-Octylcarbamoyl)-6-aminoperoxihexansäure,
N-(N'-Cyclohexylcarbamoyl)-6-aminoperoxihexansäure,
N-(N'-Phenylcarbamoyl)-6-aminoperoxihexansäure,
N-(N'-Phenylcarbamoyl)-12-aminoperoxiundecansäure und
N,N'-Bis-(6-carbamoylperoxihexansäure)-1,6-diaminohexan.

Die Vorteile des erfindungsgemäßen Verfahrens sind die kostengünstige Darstellung der Ureidocarbonsäuren, die Vermeidung eines verfahrensbedingten Salzanfalls und die Möglichkeit, die Ureidoperoxicarbonsäuren in einer "Eintopfreaktion" zu erhalten.

Die erfindungsgemäß hergestellten Ureidoperoxicarbonsäuren können als Bleichmittel für Textilien, als Oxidationsmittel oder als Desinfektionsmittel, vorzugsweise in sanitären Anlagen, verwendet werden. Zu diesen Zwecken können sie entweder in reiner Form oder in entsprechend konfektionierter Form, vorzugsweise als Granulat, eingesetzt werden. Für die Granulierung können die in EP-A-376 360, EP-A-273 334, EP-A-272 402, EP-A-256 443 oder die in den deutschen Patentanmeldungen P 40 11 185.7 und P 40 12 769.9 beschriebenen Verfahren angewendet werden.

Das erfindungsgemäße Verfahren wird anhand der folgenden Beispiele näher erläutert.

### Beispiel 1 - Herstellung der Carbamoyllactame

Das Lactam (1 Äquiv.) wird vorgelegt und unter einer Inertgas-Atmosphäre auf 80°C erwärmt. Bei einer Temperatur zwischen 75 und 85°C wird dann das entsprechende Isocyanat (1 Äquiv.) innerhalb von 60 Minuten zugetropft. Die Reaktionsmischung wird anschließend noch 3 Stunden zwischen 120 und 130°C gerührt und zum Auskristallisieren auf ein Blech gegossen. Die Ausbeuten sind quantitativ und die erhaltenen Verbindungen können meist direkt weiter umgesetzt werden.

Mit Laurinlactam wird die Reaktion in siedendem Xylol durchgeführt, wobei das Isocyanat zur Lösung des Lactams getropft wird. Die Reaktionsdauer beträgt 8 Stunden.

### Beispiel 2 - N-(N'-Phenylcarbamoyl)-6-aminoperoxihexansäure

23,2 g (0,1 Mol) N-(N'-Phenylcarbamoyl)-2-oxo-azepan werden in einer Mischung aus 50,0 g Schwefelsäure (96 gew.-%ig) und 3,6 g (0,2 Mol) Wasser gelöst und für 40 Minuten auf 60°C erwärmt. Nach dem Abkühlen auf 15°C werden 29,2 g (0,3 Mol) Wasserstoffperoxid (35 gew.-%ig) so zugetropft, daß die Innentemperatur 15°C nicht übersteigt. Die Reaktionsmischung wird unter Kühlung noch 60 Minuten zwischen 15 und 20°C gerührt und dann mit 100 ml Wasser versetzt. Die ausgefallene Peroxicarbonsäure wird abgesaugt und der Filterkuchen mit Wasser mineralsäurefrei gewaschen und bei 40°C im Wasserstrahlvakuum getrocknet.

| | |
|---|---|
| Ausbeute: | 25,6 g (96,2 %) |
| Aktivsauerstoffgehalt: | 5,81 % (96,8 %) |
| Fp. | 102°C |

### Beispiel 3 - N-(N'-Phenylcarbamoyl)-12-aminoperoxiundecansäure

31,6 g (0,1 Mol) N-Phenylcarbamoyllaurinlactam werden in 50,0 g Methansulfonsäure (98 gew.-%ig) gelöst, mit 3,6 g (0,2 Mol) Wasser versetzt und für 30 Minuten auf 80°C erwärmt. Nach Abkühlen der Reaktionsmischung auf 25°C werden 17,0 g (0,25 Mol) Wasserstoffperoxid (50 gew.-%ig) so zugetropft, daß die Innentemperatur zwischen 22 und 25°C gehalten werden kann. Nach 20 Minuten Rühren bei 25°C wird mit 100 ml Wasser versetzt und die ausgefallene Peroxicarbonsäure abgesaugt. Der Filterkuchen wird mit Wasser mineralsäurefrei gewaschen und bei 40°C im Wasserstrahlvakuum getrocknet.

| | |
|---|---|
| Ausbeute: | 34,2 g (97,7 %) |
| Aktivsauerstoffgehalt: | 4,24 % (93 %) |
| Fp.: | 116°C |

### Beispiel 4 - N-(N'-Cyclohexylcarbamoyl)-6-aminoperoxihexansäure

23,7 g (0,1 Mol) N-(N'-Cyclohexylcarbamoyl)-2-oxo-azepan werden in 50,0 g Schwefelsäure (96 gew.-%ig) gelöst, mit 3,6 g (0,2 Mol) Wasser versetzt und für 30 Minuten auf 80°C erhitzt. Nach Abkühlen der Reaktionsmischung auf 25°C werden 20,4 g (0,3 Mol) Wasserstoffperoxid (50 gew.-%ig) so zugetropft, daß die Innentemperatur 25°C nicht übersteigt. Nach 30 Minuten Rühren bei Raumtemperatur wird unter Eiskühlung mit 100 ml Wasser versetzt und die ausgefallene Peroxicarbonsäure abgesaugt. Der Filterkuchen wird mit Wasser mineralsäurefrei gewaschen und bei 40°C im Wasserstrahlvakuum getrocknet.

| | |
|---|---|
| Ausbeute: | 23,5 g (86,4 %) |
| Aktivsauerstoffgehalt: | 5,3 % (89,8 %) |
| Fp.: | 125 - 128°C |

### Beispiel 5 - N-(N'-Octylcarbamoyl)-6-aminoperoxihexansäure

26,8 g (0,1 Mol) N-(N'-Octylcarbamoyl)-2-oxo-azepan, 50,0 g Schwefelsäure (96 gew.-%ig) und 3,6 g (0,2 Mol) Wasser werden für 30 Minuten auf 80°C erwärmt und anschließend mit 20,4 g (0,3 Mol) Wasserstoffperoxid (50 gew.-%ig) für 30 Minuten bei 15°C wie in Beispiel 4 beschrieben zur Reaktion gebracht und aufgearbeitet.

| | |
|---|---|
| Ausbeute: | 29,4 g (97,3 %) |
| Aktivsauerstoffgehalt: | 5,04 % (95,3 %) |
| Fp.: | 76 - 77°C |

### Beispiel 6 - N,N'-Bis-(6-carbamoylperoxihexansäure)-1,6-diaminohexan

39,4 g (0,1 Mol) N,N'-Bis-(2-oxo-azepan-1-carbonyl)-hexandiyldiamin, 50,0 g Schwefelsäure (96 gew.-%ig) und 7,2 g (0,4 Mol) Wasser werden für 30 Minuten auf 80°C erwärmt und anschließend mit 34,0 g (0,5 Mol) Wasserstoffperoxid (50 gew.-%ig) für 30 Minuten zwischen 25 und 30°C wie in Beispiel 4 beschrieben zur Reaktion gebracht und aufgearbeitet.

| | |
|---|---|
| Ausbeute: | 43,5 g (94,2 %) |
| Aktivsauerstoffgehalt: | 5,2 % (75 %) |
| Fp.: | 155°C |

## Patentansprüche

1. Verfahren zur Herstellung von Ureidoperoxicarbonsäuren der allgemeinen Formel
A⁅NH - CO - NH - B - CO - OOH]ₓ ,
worin
x die Zahlen 1 oder 2,
A falls x = 1 ist,
Wasserstoff, C₁-C₂₀-Alkyl, Aryl, vorzugsweise Phenyl, oder Halogenaryl, vorzugsweise Chlorphenyl,
falls x = 2 ist,
C₁-C₂₀-Alkylen oder Arylen, vorzugsweise Phenylen, bedeuten,
B eine Gruppe der Formeln 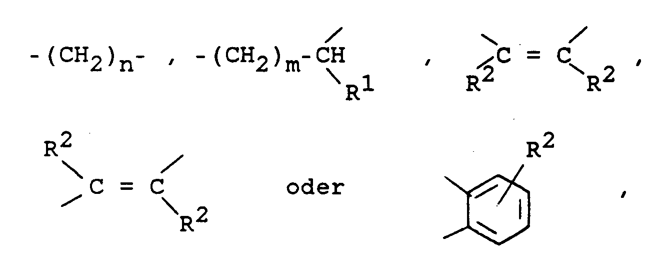 worin
n die Zahlen von 1 bis 20,
m die Zahlen 0, 1 oder 2,
R¹ C₁-C₂₀-Alkyl und
R² jeweils Wasserstoff oder C₁-C₂₀-Alkyl bedeuten,
dadurch gekennzeichnet, daß Carbamoyllactame der allgemeinen Formel 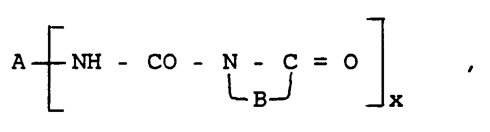 worin x, A und B die obengenannten Bedeutungen haben, in der ein- bis sechsfachen, vorzugsweise ein- bis dreifachen, Gewichtsmenge einer starken Katalysatorsäure gelöst werden, daß danach zu der Lösung des Carbamoyllactams in der Katalysatorsäure Wasser im ein- bis zehnfachen molaren Überschuß, bezogen auf das Carbamoyllactam, zugegeben und das Reaktionsgemisch zur Bildung der Ureidocarbonsäure erwärmt wird, daß anschließend eine wäßrige Wasserstoffperoxid-Lösung im ein- bis zehnfachen, vorzugsweise zwei- bis vierfachen molaren Überschuß pro oxidierbarer Carboxylgruppe der im vorhergehenden Reaktionsschritt entstandenen Ureidocarbonsäure dem Reaktionsgemisch zugesetzt wird, und daß danach die entstandene Ureidoperoxicarbonsäure aus dem Reaktionsgemisch ausgefällt wird.

2. Verfahren nach Anspruch 1, dadurch gekennzeichnet, daß als starke Katalysatorsäure Schwefelsäure, Methansulfonsäure oder ein saurer Ionenaustauscher verwendet wird.

3. Verfahren nach Anspruch 2, dadurch gekennzeichnet, daß als starke Katalysatorsäure eine 50 bis 96 gew.-%ige, vorzugsweise eine 75 bis 96 gew.-%ige, wäßrige Schwefelsäure-Lösung eingesetzt wird.

4. Verfahren nach Anspruch 1, dadurch gekennzeichnet, daß als starke Katalysatorsäure eine 80 bis 98 gew.-%ige Methansulfonsäure verwendet wird.

5. Verfahren nach einem der Ansprüche 1 bis 4, dadurch gekennzeichnet, daß zu der Lösung des Carbamoyllactams in der Katalysatorsäure Wasser im ein- bis dreifachen molaren Überschuß, bezogen auf das Carbamoyllactam, zugegeben wird.

6. Verfahren nach einem der Ansprüche 1 bis 5, dadurch gekennzeichnet, daß das aus dem Carbamoyllactam, der Katalysatorsäure und Wasser bestehende Reaktionsgemisch für mindestens 10 Minuten auf mindestens 40°C erwärmt wird.

7. Verfahren nach einem der Ansprüche 1 bis 6, dadurch gekennzeichnet, daß Wasserstoffperoxid als 5 bis 95 gew.-%ige wäßrige Lösung dem Reaktionsgemisch zugesetzt wird.

8. Verfahren nach einem der Ansprüche 1 bis 7, dadurch gekennzeichnet, daß man Wasserstoffperoxid mindestens 15 Minuten auf das die Ureidocarbonsäure enthaltene Reaktionsgemisch einwirken läßt.

9. Verfahren nach einem der Ansprüche 1 bis 8, dadurch gekennzeichnet, daß man die erhaltene Ureidoperoxicarbonsäure in ein Granulat zur Verwendung als Bleich-, Oxidations- oder Desinfektionsmittel einarbeitet.

## Claims

1. A process for the preparation of a ureidoperoxycarboxylic acid of the formula
A⁅NH - CO - NH - B - CO - OOH]ₓ ,
in which
x is the number 1 or 2,
A, if x is 1,
is hydrogen, C₁-C₂₀-alkyl, aryl, preferably phenyl, or haloaryl, preferably chlorophenyl, or if x is 2,
is C₁-C₂₀-alkylene or arylene, preferably phenylene,
B is a group of the formula 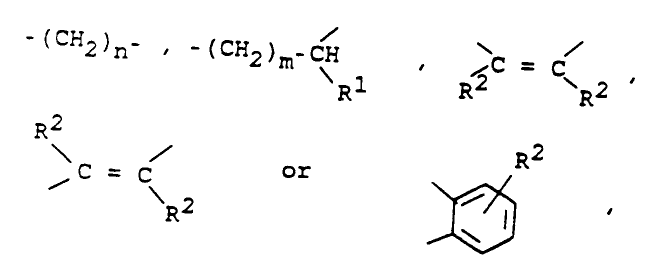 in which
n is a number from 1 to 20,
m is the number 0, 1 or 2,
R¹ is C₁-C₂₀-alkyl and
R² is in each case hydrogen or C₁-C₂₀-alkyl,
which comprises dissolving carbamoyllactams of the formula 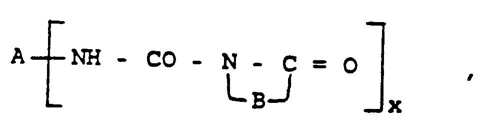 in which x, A and B have the abovementioned meanings, in a one- to six-fold, preferably a one- to three-fold, amount by weight of a strong catalyst acid, by then adding water in a one- to ten-fold molar excess, relative to the carbamoyllactam, to the solution of the carbamoyllactam in the catalyst acid and by heating the reaction mixture to form the ureidocarboxylic acid, by then adding to the reaction mixture an aqueous hydrogen peroxide solution in a one- to ten-fold, preferably a two- to four-fold, molar excess per oxidizable carboxyl group of the ureidocarboxylic acid formed in the preceding reaction step, and by then precipitating the resulting ureidoperoxycarboxylic acid from the reaction mixture.

2. The process as claimed in claim 1, wherein sulfuric acid, methanesulfonic acid or an acidic ion exchanger is used as the strong catalyst acid.

3. The process as claimed in claim 2, wherein a 50 to 96% strength by weight, preferably a 75 to 96% strength by weight, aqueous sulfuric acid solution is employed as the strong catalyst acid.

4. The process as claimed in claim 1, wherein an 80 to 98% strength by weight methanesulfonic acid is used as the strong catalyst acid.

5. The process as claimed in one of claims 1 to 4, wherein water is added to the solution of the carbamoyllactam in the catalyst acid in a one- to three-fold molar excess, relative to the carbamoyllactam.

6. The process as claimed in one of claims 1 to 5, wherein the reaction mixture composed of the carbamoyllactam, the catalyst acid and water is heated to at least 40°C for at least 10 minutes.

7. The process as claimed in one of claims 1 to 6, wherein hydrogen peroxide is added to the reaction mixture as a 5 to 95% strength by weight aqueous solution.

8. The process as claimed in one of clams 1 to 7, wherein hydrogen peroxide is allowed to act on the reaction mixture containing the ureidocarboxylic acid for at least 15 minutes.

9. The process as claimed in one of claims 1 to 8, wherein the ureidoperoxycarboxylic acid obtained is incorporated into granules for use as a bleach, oxidant or disinfectant.

## Revendications

1. Procédé pour la préparation d'acides uréidoperoxycarboxyliques de formule générale :
A⁅NH - CO - NH - B - CO - OOH]ₓ ,
x vaut 1 ou 2,
A si x = 1,
représente l'hydrogène, les alkyle en C₁-C₂₀, aryle, de préférence phényle, ou halogénoaryle, de préférence le chlorophényle,
si x = 2,
les alkylène en C₁-C₂₀ ou arylène, de préférence le phénylène,
B représente un groupe de formules : 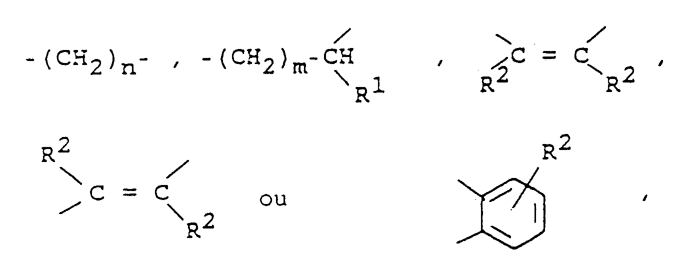 où
n va de 1 à 20,
m vaut 0, 1 ou 2,
R¹ représente les alkyle C₁-C₂₀ et
R² représente chaque fois l'hydrogène ou les alkyle en C₁-C₂₀,
caractérisé en ce qu'on dissout des carbamoyl-lactames de formule générale : 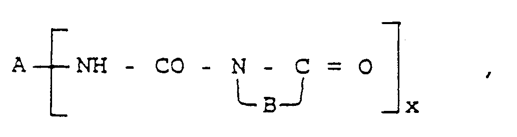 dans laquelle x, A et B ont les significations données ci-dessus dans une quantité 1 à 6 fois, de préférence 1 à 3 fois, d'un acide catalytique fort, en ce qu'on ajoute de l'eau à la solution du carbamoyl-lactame dans l'acide catalytique, dans un excès 1 à 10 fois molaire, par rapport au carbamoyl-lactame et on chauffe le mélange réactionnel pour former l'acide uréidocarboxylique, en ce qu'on ajoute ultérieurement une solution aqueuse de peroxyde d'hydrogène dans un excès 1 à 10 fois molaire, de préférence 2 à 4 fois molaire, par groupe carboxyle oxydable de l'acide uréidocarboxylique formé dans l'étape réactionnelle précédente, et en ce qu'on précipite du mélange réactionnel l'acide uréidoperoxycarboxylique formé.

2. Procédé selon la revendication 1, caractérisé en ce qu'on utilise en tant qu'acide catalytique fort l'acide sulfurique, l'acide méthanesulfonique et un échangeur d'ions acide.

3. Procédé selon la revendication 2, caractérisé en ce qu'on utilise en tant qu'acide catalytique fort une solution aqueuse d'acide sulfurique à 50 à 96 % en poids, de préférence de 75 à 96 %.

4. Procédé selon la revendication 1, caractérisé en ce qu'on utilise en tant qu'acide catalytique fort l'acide méthanesulfonique à 80 à 98 % en poids.

5. Procédé selon une des revendications 1 à 4, caractérisé en ce qu'on ajoute de l'eau à la solution du carbamoyl-lactame dans l'acide catalytique, dans un excès 1 à 3 fois molaire par rapport au carbamoyllactame.

6. Procédé selon une des revendications 1 à 5, caractérisé en ce qu'on chauffe le mélange réactionnel comportant le carbamoyl-lactame, l'acide catalytique et l'eau, pendant au moins 10 minutes, à une température de 40 °C.

7. Procédé selon une des revendications 1 à 6, caractérisé en ce qu'on ajoute au mélange réactionnel du peroxyde d'hydrogène sous forme de solution aqueuse de 5 à 95 % en poids.

8. Procédé selon une des revendications 1 à 7, caractérisé en ce qu'on fait agir le peroxyde d'hydrogène pendant au moins 15 minutes sur le mélange réactionnel contenant l'acide uréidocarboxylique.

9. Procédé selon une des revendications 1 à 8, caractérisé en ce qu'on incorpore l'acide uréidoperoxycarboxylique dans un granulat pour utilisation en tant qu'agent blanchissant, agent d'oxydation ou désinfectant.
